# EUROPÄISCHE PATENTSCHRIFT

(11) **EP 0 425 587 B1**
(45) Veröffentlichungstag und Bekanntmachung des Hinweises auf die Patenterteilung: **08.06.1994**
(21) Anmeldenummer: 89913044.7
(22) Anmeldetag: 24.11.1989
(51) Int. Cl.: C12Q 1/04, C12M 1/34

(54) **VERFAHREN ZUR FESTSTELLUNG BIOLOGISCHER AKTIVITÄTEN IN EINER PROBE UND VORRICHTUNG ZUR DURCHFÜHRUNG DES VERFAHRENS**
PROCESS FOR DETERMINING BIOLOGICAL ACTIVITIES IN A SAMPLE AND A DEVICE FOR IMPLEMENTING IT
PROCEDE POUR LA MISE EN EVIDENCE D'ACTIVITES BIOLOGIQUES DANS UN ECHANTILLON ET DISPOSITIF POUR LA MISE EN OEUVRE DE CE PROCEDE

(30) Priorität: 12.05.1989 AT 1147/89
(43) Veröffentlichungstag der Anmeldung: 08.05.1991
(73) Patentinhaber: AVL Medical Instruments AG, 8207 Schaffhausen (CH)
(72) Erfinder: KARPF, Hellfried, A-8010 Graz (AT); SMOLE, Herbert, CH-8253 Diessenhofen (CH)
(74) Vertreter: Krause, Walter, Dr. Dipl.-Ing.
(86) Internationale Anmeldenummer: AT8900110
(87) Internationale Veröffentlichungsnummer: WO9013663

(56) Entgegenhaltungen:
- EP-A- 105 870
- EP-A- 333 253
- WO-A-82/04264
- WO-A-90/14414
- DE-B- 2 632 556
- PATENT ABSTRACTS OF JAPAN, vol. 7, no. 64 (P-183)(1209), 17 March 1983#

## Beschreibung

Erfindung betrifft ein Verfahren zur Feststellung biologischer Aktivitäten in einer Probe, wobei die Probe und eine Nährlösung in einen verschließbaren Behälter gefüllt und Bedingungen ausgesetzt werden, welche bei Vorliegen von Mikroorganismen in der Probe metabolische Prozesse ermöglichen, wobei die Konzentration von Ausgangsstoffen vermindert und jene von Stoffwechselprodukten erhöht wird und eine Vorrichtung zur Durchführung des Verfahrens.

Für viele Anwendungsgebiete ist es wichtig, rasch feststellen zu können, ob eine Probe mit Mikroorganismen, beispielsweise mit Bakterien, kontaminiert ist, wobei hier vor allem der medizinische Bereich, die pharmazeutische Industrie, die Lebensmittelindustrie sowie der Umweltschutz zu nennen wäre. Der Begriff "Probe" soll hier sehr umfassend gedeutet werden und vor allem folgende Substanzen beinhalten: Festes und flüssiges biologisches Material (z.B. Blut), Lebensmittelproben, wie Tiefkühlkost und Konserven, Verpackungsmaterial, klinische Instrumente und Laborgeräte bzw. von deren Oberflächen entnommene Proben, medizinisches Gerät, Hilfs- und Verbandsstoffe sowie Erdproben und Wasserproben, insbesondere Trinkwasserproben.

Seit langem sind rein manuelle Methoden bekannt, bei welchen die zu untersuchende Probe einem Kulturgefäß mit einer Nährlösung zugesetzt wird, wobei dann in bestimmten Abständen rein visuell das Wachstum der Kultur beobachtet wird, um daraus auf die Art oder das Vorhandensein eines Mikroorganismus zu schließen.

Es sind auch bereits einige technische Verfahren bzw. Vorrichtungen bekannt, mit welchen die von Mikroorganismen verursachten biologischen Aktivitäten in einer Probe festgestellt werden können, wobei z.B. das durch den Metabolismus der Mikroorganismen entstehende CO₂, bzw. die Anderung des CO₂-Gehaltes als Meßwert für die Bestimmung der biologischen Aktivität herangezogen wird.

So ist es beispielsweise bekannt, die zu untersuchende Probe zusammen mit einer radioaktiv markierten Nährlösung in einen Behälter einzuschließen und die sich über der Nährlösung befindliche Atmosphäre auf das Vorhandensein radioaktiver Gase zu untersuchen, woraus auf Mikroorganismen in der Probe geschlossen werden kann.

Meßsysteme dieser Art sind beispielsweise aus der US-PS 3 676 679 oder 3 935 073 bekannt. Obwohl solche Meßsysteme rasch und zuverlässig arbeiten, haben sie den Nachteil, daß radioaktive Substanzen gehandhabt werden müssen und es notwendig ist, kontinuierlich Proben aus dem Gasraum über der Nährlösung zu entnehmen und zu vermessen. Bei der Entnahme der Proben aus dem Gasraum kann es durch das Entnahmeorgan leicht zu Kontaminationen der übrigen zu vermessenden Proben kommen, wodurch es leicht zu Falschaussagen kommen kann.

Aus der EP-A 0 158 497 ist es weiters bekannt, die biologische Aktivität einer Probe mit Hilfe der Infrarot-Absorption zu bestimmen. Dabei wird einem geschlossenen Behälter, welcher eine Nährlösung enthält, eine Probe zugesetzt, welche auf das Vorliegen von Mikroorganismen untersucht wird. Der Behälter wird dann bestimmten Bedingungen ausgesetzt, insbesondere werden bestimmte Temperaturen über festgelegte Zeiträume eingehalten, welche den Metabolismus der Mikroorganismen ermöglichen, wobei im Gasraum über der Nährlösung unter Umwandlung der Kohlenstoffquelle CO₂ gebildet wird. Aus dem Gasraum wird dann eine Probe entnommen und einer Meßzelle zugeführt, wo mittels Infrarot-Absorption der CO₂-Gehalt gemessen wird. Auch hier besteht das Problem der Kontamination nachfolgender Proben, wobei als weiterer Nachteil anzuführen ist, daß die Infrarot-Absorptionsmessung weniger sensitiv ist als die Meßmethode mit radioaktiver Markierung.

Um das Problem der Kreuzkontamination zu vermeiden, wird in der EP-A 0 104 463 ein Verfahren sowie eine Vorrichtung der eingangs genannten Art vorgeschlagen, welche ebenfalls auf der Basis der Infrarot-Absorptionsmessung des durch metabolische Prozesse entstehenden CO₂ beruht. Dabei erfolgt jedoch keine Probennahme, sondern Infrarotstrahlung wird direkt durch die Behälterwand in den Gasraum über der Nährlösung geleitet und deren Absorption gemessen. Durch diese nichtinvasive Meßmethode können zwar Kreuzkontaminationen weitgehend ausgeschlossen werden; nachteilig bei dieser Methode ist jedoch die nach wie vor geringere Sensitivität gegenüber radiometrischen Verfahren, sowie die Tatsache, daß das Meßergebnis durch andere Gaskomponenten, welche im gleichen Frequenzband wie CO₂ absorbieren, verfälscht wird. Als Beispiel wären hier vor allem die Absorptionsbanden von Wasserstoffdampf zu nennen. Die verwendeten Probenbehälter müssen in einem relativ engen Frequenzbereich durchlässig sein, sodaß nur bestimmte Behältermaterialien in Frage kommen. Ein zusätzlicher Nachteil besteht darin, daß die Erzeugung bzw. Filterung der benötigten Infrarotstrahlung relativ aufwendig und teuer ist.

Weiters sind aus der EP-A 0 333 253 und aus der WO 90/14414 ähnliche Verfahren und Vorrichtungen bekannt, welche kontinuierliche Messungen von durch Mikroorganismen gebildete Metabolite mittels einer oder mehrerer Fluoreszenzsensoren ermöglichen, wobei die Sensoren in direktem Kontakt mit den, in einem verschließbaren Behälter befindlichen zu messenden Stoff stehen. In der EP-A 0 333 253 werden auf diese Art Änderungen des pH-Wertes oder der CO₂-Konzentration gemessen. Gleiches gilt für die WO 90/14414.

Aufgabe der Erfindung ist es, ein Verfahren bzw. eine Vorrichtung zur Feststellung biologischer Aktivitäten in einer Probe vorzuschlagen, welches zumindest dieselbe Sensitivität wie radiometrische Verfahren aufweist, wobei es wünschenswert ist, nähere Angaben über die Art der Mikroorganismen zu gewinnen und Kreuzkontaminationen zu vermeiden sowie ein billiges, einfaches Meßverfahren zu realisieren.

Diese Aufgabe wird erfindungsgemäß dadurch gelöst, daß die Konzentration zumindest eines durch metabolische Prozesse umsetzbaren (gewonnenen oder verbrauchten] Stoffes kontinuierlich gemessen wird und bei Änderung der Konzentration um einen vorgebbaren Schwellwert die Konzentrationsänderung zumindest eines weiteren Stoffes gemessen wird, wobei mittels Optoden, welche in direktem Kontakt mit den zu messenden Stoffen stehen ein Meßsignal erzeugt wird, aus dessen zeitlicher Änderung auf das Vorliegen von Mikroorganismen geschlossen wird. Die in Massenfertigung billig herstellbaren optischen Sensoren (Optoden), welche mit der zu messenden Substanz in direktem Kontakt stehen, ermöglichen kontinuierliche Messungen in abgeschlossenen Systemen, wobei für jede Probe neue Optoden verwendet werden und so Kreuzkontaminationen ausgeschlossen werden. Beispielsweise kann im Minimalfall bereits ein Zwei fach-Sensor (Bisensor) ausreichen, um anhand der relativen Anderungen der Konzentrationen der erfaßten Stoffe gegenüber den Ausgangskonzentrationen eine Aussage über die Art der Mikroorganismen zu treffen.

Erfindungsgemäß ist vorgesehen, daß die Konzentration zumindest zweier Stoffe aus der Gruppe CO₂, O₂, H⁺(pH), NH₄⁺, H₂S und H₂ gemessen wird, wobei die Indikatorsubstanz der Optoden auf eine Änderung der Konzentration der Stoffe mit einer Änderung ihres Lumineszenz-, Absorptions- oder Reflexionsverhaltens reagiert, bzw. daß die Konzentration zumindest zweier Stoffe aus der Gruppe CO₂, O₂, H⁺(pH) und NH₄⁺ gemessen wird, wobei die Indikatorsubstanz der Optoden auf eine Änderung der Konzentration der Stoffe mit einer Änderung der Lumineszenzabklingzeit der emittierten Lumineszenzstrahlung reagiert. Es können somit alle, in metabolischen Prozessen eine wesentliche Rolle spielenden Stoffe durch zumindest ein optisches Meßprinzip erfaßt werden.

Insbesondere ist entsprechend der Erfindung bei der Messung von z.B. Blutproben vorgesehen, daß die CO₂-Konzentration kontinuierlich gemessen und daß nach deren Anstieg um 0,1 bis 10% über einen minimalen Wert die Änderung der O₂-Konzentration und die Änderung des pH-Wertes bestimmt wird.

Es ist entsprechend der Erfindung auch möglich, die O₂-Konzentration kontinuierlich zu messen und nach deren Abfall um 0,1 bis 10% von einem maximalen Wert die Änderung der CO₂-Konzentration und die Änderung des pH-Wertes zu bestimmen. Das hat den Vorteil, daß bei der O₂-Konzentration bereits zu früheren Zeitpunkten signifikante Änderungen meßbar sind.

Entsprechend der untenstehenden Tabelle kann dabei nach einer Änderung der CO₂ -Konzentration um 0,1 bis 10% äußerst rasch eine Entscheidungshilfe bei der Beurteilung der in Frage kommenden Bakterienstämme angeboten werden, wobei zumindest eine Einschränkung auf wenige Spezies möglich ist.

| Gruppe | O₂-Änderung | pH-Änderung | Gram |
|---|---|---|---|
| Enterobakteriaceae | ↓ | ↓ | neg. |
| Pseudomonas Spezies | ↓↓ | +/-o | neg. |
| Acinetobacter Spezies | ↓↓ | ↓ | neg. |
| Bakteroide Spezies | +/-o | ↓ | neg. |
| Staphylokokkus | ↓↓ | ↓↓ | pos. |
| Staphylokokkus epidermidis | ↓ | ↓ | pos. |
| Streptokokkus faecalis | ↓↓ | ↓↓ | pos. |
| Streptokokkus pyogenes | ↓ | ↓↓ | pos. |
| Streptokokkus pneumoniae | ↓ | ↓↓ | pos. |
| Candida albicans | ↓ | ↓↓ | pos. |
| Chlostridium perfringens | +/-o | ↓↓ | pos. |

Für die Änderung der O₂-Konzentration bedeutet schwach fallend (↓) eine Änderung im Bereich von 3 bis 21% des Ausgangs- bzw. Maximalwertes und stark fallend (↓↓) eine Änderung über 21%. Bei der pH-Änderung liegt die Grenze zwischen schwach fallendem (↓) und stark fallendem (↓↓) pH-Wert bei 0,15% des Ausgangswertes.

Beispielsweise kann bei schwach fallender O₂ -Konzentration und schwach fallendem pH-Wert (zu sauren Werten) auf das Vorliegen von Enterobakterien oder auf das Vorhandensein von Staphylokokkus epidermidis geschlossen werden.

Erfindungsgemäß kann zusätzlich die Gramfärbung der Probe auf bekannte Art festgestellt werden, wobei bei negativer Gramfärbung auf Enterobakterien und bei positiver Gramfärbung auf Staphylokokkus epidermidis geschlossen wird.

Weiters lassen sich durch die in der Tabelle vermerkten Änderungen der O₂-Konzentration und des pH-Wertes die angeführten Spezies unterscheiden.

Weiters ist es möglich, in einen verschließbaren Behälter eine eine Kohlenstoff-Verbindung enthaltende Nährlösung zu füllen und der Nährlösung ein auf die Änderung des CO₂-Gehaltes mit einer Änderung des Fluoreszenzverhaltens reagierenden Fluoreszenzindikator zuzusetzten. In den Behälter wird dann eine Blutprobe eingebracht, wobei bei Vorliegen von Mikroorganismen in der Probe metabolische Prozesse ermöglicht werden, bei welchen CO₂ produziert wird. Der Inhalt des Behälters wird mit Anregungsstrahlung beaufschlagt und die vom Fluoreszenzindikator emittierte Strahlung gemessen, wobei aus einer Änderung des Fluoreszenzverhaltens auf das Vorliegen von Mikroorganismen geschlossen wird. Zum Beispiel können aus der DE-A 23 60 384 bekannt gewordene Indikatorkapseln bzw. indikatorhältige Mikrokapseln, deren Kapselwände beispielsweise aus polymerisierten hydrophilen Monomeren bestehen und Durchmesser von 20 - 200 nm aufweisen, zugesetzt werden.

Eine erfindungsgemäße Vorrichtung zur Feststellung biologischer Aktivitäten in einer Probe, wobei ein verschließbarer Behälter vorgesehen ist, welcher eine Nährlösung enthält und zur Aufnahme der Probe dient, wobei Einrichtungen vorgesehen sind, die bei Vorliegen von Mikroorganismen in der Probe metabolische Prozesse ermöglichen, ist dadurch gegeben, daß mehrere Optoden im Inneren des verschließbaren Behälters zur gleichzeitigen Bestimmung mehrerer durch den metabolischen Prozeß in deren Konzentration veränderbarer Stoffe vorgesehen sind, sowie daß eine jeder Optode zugeordnete Anregungs- und Detektionseinrichtung vorgesehen ist, mit welcher eine Auswerteeinrichtung zur Feststellung der zeitlichen Änderung der Konzentration der Stoffe verbunden ist. Dabei sind unterschiedliche Kombinationen von jeweils zwei Optoden (z.B. O₂ und pH) zu einem Bisensor oder von jeweils drei Optoden (CO₂, O₂ und pH) zu einem Trisensor von Vorteil.

Erfindungsgemäß sind Optoden zur selektiven Erfassung zumindest zweier im metabolischen Prozeß als Ausgangs-, Zwischen- oder Endprodukt vorliegender Stoffe aus der Gruppe CO₂, O₂, H₂, H⁺(pH), NH₄⁺ und H₂S, vorhanden.

Dabei kann die Anregungs- und Detektionseinrichtung eine Lichtquelle und einen Detektor sowie einen vorzugsweise zwei armigen Lichtleiter zur Zufuhr der Anregungsstrahlung zur Optode bzw. den Optoden und zur Abfuhr des optischen Signals zum Detektor aufweisen.

Eine vorteilhafte Ausgestaltung der Erfindung sieht vor, daß die Optoden zu einem Multilayer-Sensor zusammengefaßt sind.

In einer einfachen Ausführungsvariante sind die Optoden an der Innenwand eines optisch durchlässigen Behälters fixiert und über deren direkt an die Außenwand des Behälters heranführbare Anregungs- und Detektionseinrichtung mit der Auswerteeinrichtung verbunden. Die in großen Stückzahlen bereits sehr billig herstellbaren Optoden können bei dieser Ausführungsvariante dirket auf die Innenwand des Probenbehälters aufgeklebt werden, welcher bereits mit Nährlösung gefüllt und versiegelt gelagert werden kann. Nach Zugabe der Probe, beispielsweise einer Blutprobe, wird der Behälter die für das Wachstum der Kultur notwendige Zeit thermostatisiert und ggf. geschüttelt, wonach die Konzentration des durch metabolische Prozesse umsetzbaren Stoffes beispielsweise über einen an die Außenwand des Behälters heranführbaren optischen Lichtleiter gemessen wird.

Erfindungsgemäß können zumindest die Optoden im Gasraum des zumindest teilweise optisch durchlässigen Behälters über der mit der Probe versetzten Nährlösung angeordnet sein und die Anderung der Konzentration zumindest eines gasförmigen Metaboliten messen.

Weiters ist es gemäß einer weiteren Ausführungsvariante auch vorgesehen, daß die Optoden an einem den Behälter verschließenden, optisch durchlässigen Stöpsel angeordnet sind.

Es ist bei dieser Methode jedoch auch möglich, daß die Optoden in einem von der mit der Probe versetzten Nährlösung bedeckten Bereich des Behälters, ggf. am Boden des Behälters, angeordnet sind und die Änderung der Konzentration zumindest eines Stoffes in der Nährlösung messen. Letztere Anordnung zeichnet sich dadurch aus, daß der Metabolit praktisch am Orte seines Entstehens gemessen wird, wodurch die Aussage ob eine Kultur positiv oder negativ ist, viel rascher getroffen werden kann.

Eine besonders vorteilhafte Ausgestaltung der Erfindung sieht vor, daß eine Einrichtung zur Thermostatisierung der Probe vorgesehen ist, in welcher in markierten Positionen mehrere Behälter gleichzeitig angeordnet sind, wobei jedem der Behälter eine Anregungs- und Detektionseinrichtung zugeordnet ist, welche die in jedem Behälter angeordneten Optoden mit Anregungsstrahlung versorgt und das resultierende optische Signal detektiert, sowie daß die Signale der Detektionseinrichtung samt einem Positionserkennungssignal der Auswerteeinrichtung zuführbar sind. Die Thermostatisiereinrichtung kann als thermostatisierbare Trägerplatte beispielsweise schachbrettartig ausgebildet sein, wodurch eine große Anzahl von Kulturbehältern, beispielsweise bis zu 600 Stück, gleichzeitig vermessen werden kann. Vorteilhafterweise ist gegenüber herkömmlichen Geräten dieser Art nach dem Einbringen der Probe in die einzelnen Behälter keinerlei Handhabung mehr notwendig, da Brutvorgang und kontinuierliche Messung in einem Gerät vollkommen automatisch erfolgen. Durch die kontinuierliche Messung ist im Gegensatz zu herkömmlichen Meßverfahren, wo die einzelnen Kulturgefäße händisch ein- bis zweimal am Tag in eine Auswerteeinheit eingesetzt werden, verzögerungsfrei der Zeitpunkt feststellbar, ab wann eine Kultur positiv wird. Ab diesem Zeitpunkt können weitere im Stoffwechsel involvierte Stoffe optisch gemessen und die Art der Mikroorganismen bestimmt werden. Es steht somit ein hochsensitives automatisches Meßverfahren zur Verfügung, mit welchem nichtinvasive, kontinuierliche Messungen möglich sind. Die Auswerteeinheit kann entweder selbst einen Mikrocomputer beinhalten, welcher den Status der einzelnen Behälter anzeigt oder über ein Interface mit einem Computer verbunden sein.

Eine andere Ausführungsvariante der Erfindung sieht vor, daß eine Einrichtung zur Thermostatisierung der Probe vorgesehen ist, welche mehrere Behälter gleichzeitig aufnimmt, sowie daß ein Zuführmechanismus oder Probenwechsler vorgesehen ist, welcher die einzelnen Behälter automatisch einem Meßplatz zuführt, in welchem die in jedem Behälter angeordneten Optoden mit der Anregungs- und Detektionseinrichtung in optischen Kontakt treten. Während die oben beschriebene Ausführungsvariante gänzlich ohne bewegliche Teile auskommt, beinhaltet diese Variante einen Probenwechsler herkömmlicher Art, welcher die einzelnen Proben automatisch einem Meßplatz zuführt. Der Vorteil dieser Anordnung besteht darin, daß der elektronische bzw. elektrooptische Aufwand der Anordnung geringer gehalten werden kann.

Des weiteren ist es erfindungsgemäß möglich, daß die Optoden an der Spitze einer in den Behälter einführbaren Sonde befestigbar sind, welche Sonde Lichtleiteinrichtungen der Anregungs- und Detektionseinrichtung aufnimmt. Beispielsweise kann die Sonde durch eine mit einem Septum verschlossene Öffnung des Behälters in die mit der Probe versetzte Nährlösung oder in den Gasraum darüber eingebracht werden.

Eine weitere Ausführungsvariante der Erfindung sieht vor, daß der Behälter mit einem Septum verschlossen ist, welches von der Hohlnadel eines Probennahmegefäßes durchstechbar ist, daß im Probennahmegefäß Optoden zur gleichzeitigen Bestimmung mehrerer durch den metabolischen Prozeß in deren Konzentration veränderbarer Stoffe angeordnet sind, daß nach dem Einbringen der Probe in den die Nährlösung enthaltenden Behälter über die Hohlnadel des Probennahmegefäßes eine Strömungsverbindung vom Gasraum des Behälters zu den Optoden besteht, sowie daß eine jeder Optode zugeordnete Anregungs- und Detektionseinrichtung vorgesehen ist, mit welcher eine Auswerteeinrichtung zur Feststellung der zeitlichen Änderung der Konzentration der Stoffe verbunden ist. Das Probennahmegefäß kann beispielsweise als evakuiertes Gefäß ausgeführt sein, an dessen Innenwand eine CO₂- und eine O₂-Optode fixiert sind. Die Probe, beispielsweise Blut, wird durch das anliegende Vakuum in den Behälter gesogen. Mit der Nadel wird dann das Septum des Behälters mit der Nährlösung durchstochen, wobei das Blut in das Kulturgefäß eingebracht wird. Über die Hohlnadel gelangen nun CO₂ und O₂ aus dem Gasraum über der Nährlösung zum Sensor, wo sie gemessen werden. Kulturbehälter und Probennahmegefäß sind vorzugsweise als Einwegartikel konzipiert, welche nach Verwendung weggeworfen werden.

Die Erfindung wird im folgenden anhand von Zeichnungen näher erläutert. Es zeigen:
- Figur 1: eine erfindungsgemäße Vorrichtung in schematischer Darstellung,
- Figuren 2a, 2b, 3 und 4: Ausführungsvarianten der Vorrichtung nach Fig. 1,
- Figur 5: eine automatisch messende Ausführungsvariante zur gleichzeitigen Messung mehrerer Behälter,
- Figur 6 und 7: Ausführungsvarianten nach Fig. 5 im Detail, sowie
- Figur 8 bis 10: Diagramme von Meßkurven.

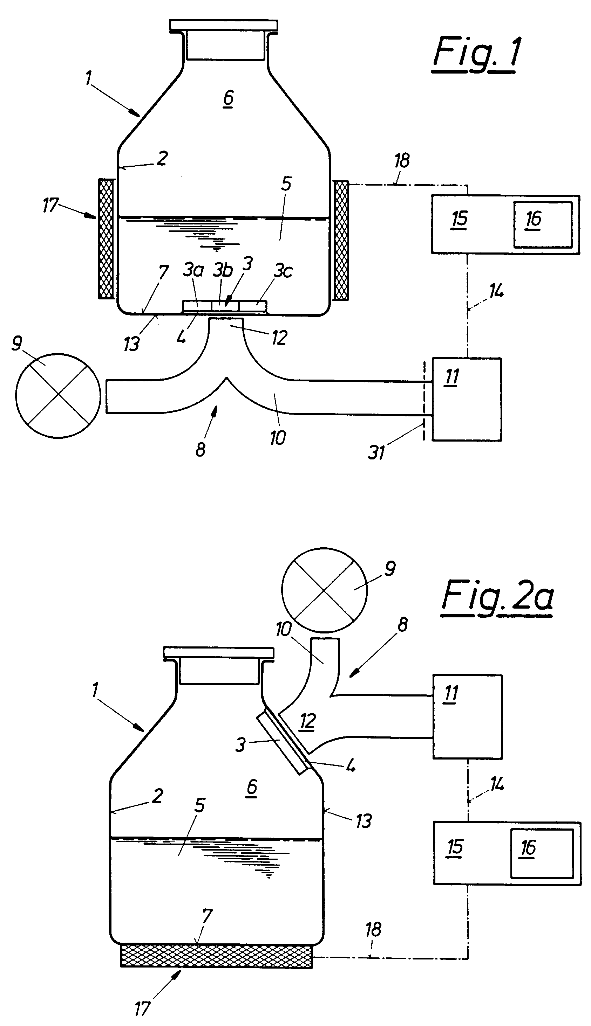

Die in Fig. 1 dargestellte Vorrichtung zur Feststellung biologischer Aktivitäten in einer Probe, weist einen verschließbaren, optisch durchlässigen Behälter 1 auf, an dessen Innenwand 2 eine Optode 3, beispielsweise mit einer optisch durchlässigen Kleberschicht 4, befestigt ist.

In der Optode 3 sind zwei oder mehrere Einzeloptoden 3a, 3b und 3c zu einem Multilayer-Sensor zusammengefaßt, sodaß gleichzeitig, beispielsweise die Konzentrationsänderungen von O₂ und CO₂ sowie die Änderung des pH-Wertes festgestellt werden können. Die einzelnen Optoden 3a, bis 3c bzw. deren Indikatorsubstanzen können auch schichtweise übereinander angeordnet oder in einer Polymermembran homogen verteilt eingebettet sein. Die Kombination einer CO₂- und einer O₂-Optode zu seinem Sensor ist beispielsweise aus der EP-A 105 870 bekannt geworden.

Anstelle der in den folgenden Ausführungsvarianten mit 3 bezeichneten Optode können Optoden zur Messung von O₂, CO₂, H⁺ (pH), NH₄⁺, H₂S und H₂, bzw. eine für die jeweilige Meßsituation erforderliche Kombination dieser Optoden angeordnet sein.

Im Behälter 1 befindet sich die Nährlösung 5 mit z.B. einer Kohlenstoffverbindung (Glucose), welche durch metabolische Prozesse von in der Probe vorliegenden Mikroorganismen in ein Stoffwechselprodukt, beispielsweise in CO₂ umgewandelt wird, wobei gleichzeitig z.B. O₂ verbraucht wird und sich der pH-Wert ändert. Dadurch ändert sich im Gasraum 6 über der Nährlösung 5, sowie in der Nährlösung selbst die Konzentration des Stoffwechselproduktes und der Ausgangsstoffe, welche mit den Optoden 3a, 3b, 3c, welche in Fig. 1 am Boden 7 des Behälters 1 angeordnet sind, gemessen wird. Die Anregungs- und Detektionseinrichtung 8 besteht aus einer Lichtquelle 9, einem Detektor 11 sowie einem zweiarmigen Lichtleiter 10, dessen einer Arm mit der Lichtquelle und dessen anderer Arm mit dem Detektor 11 in Verbindung steht. Das Ende 12 des Lichtleiters liegt direkt an der Außenwand 13 des Behälters an und versorgt die Optoden 3a, 3b, 3c durch die optisch durchlässige Behälterwand mit Anregungsstrahlung und empfängt gleichzeitig das optische Signal, beispielsweise die von den Optoden emittierte Fluoreszenzstrahlung.

Durch entsprechende Filtereinrichtungen 31, beispielsweise ein Filterrad, vor dem Detektor 11 kann dafür gesorgt werden, daß die entsprechenden Signale den jeweiligen Optoden 3a, 3b, 3c zugeordnet werden können.

Die Detektorsignale werden über eine Leitung 14 einer Auswerteeinrichtung 15 zugeführt, in welcher die zeitliche Änderung beispielsweise des CO₂-Gehaltes festgestellt und der Status der Probe über ein Display 16 angezeigt wird.

Die für den Ablauf der metabolischen Prozesse notwendigen Bedingungen im Behälter werden mit Hilfe der Einrichtung 17 aufrecht erhalten, wobei die Einrichtung 17 vor allem für die richtige Thermostatisierung der Probe verantwortlich ist und über eine Steuerleitung 18 mit der Auswerteeinrichtung 15 verbunden ist.

Anstelle der Einrichtung 17 kann in Fig. 1 und allen folgenden Ausführungsvarianten auch eine Luftheizung zur Thermostatisierung der Proben verwendet werden.

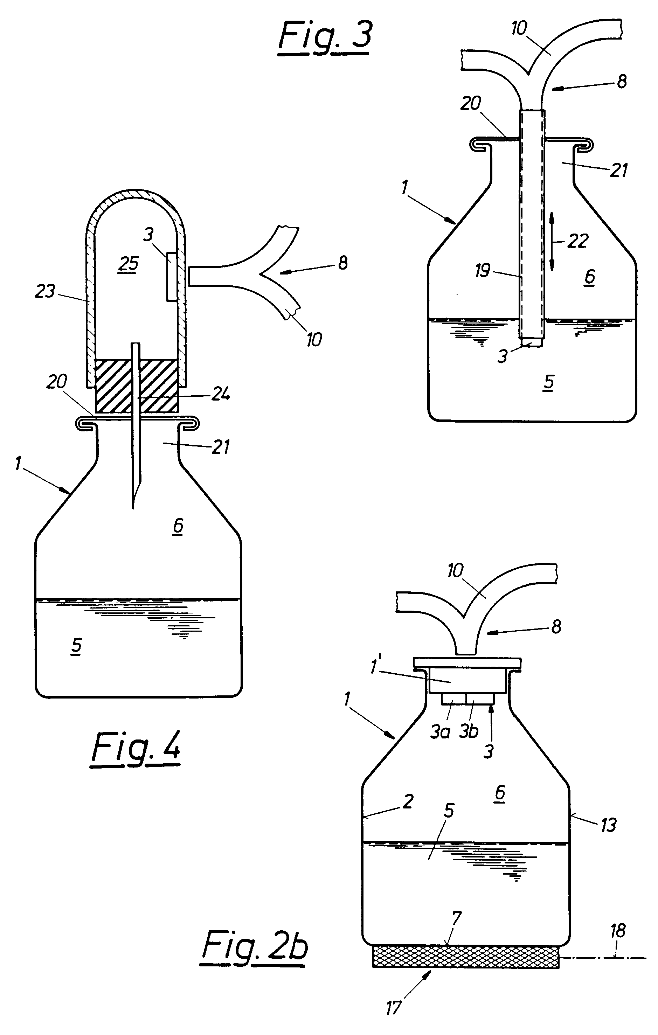

Die in Fig. 2a dargestellte Ausführungsvariante unterscheidet sich von jener nach Fig. 1 nur dadurch, daß die Optode 3 im Gasraum 6 des Behälters 1 angeordnet ist und nur gasförmige Metaboliten gemessen werden können. Die Thermostatisierung erfolgt hier über den Boden 7 des Behälters 1. Entsprechend einer Ausführungsvariante nach Fig. 2b kann die Optode 3, bzw. Optoden 3a, 3b, an einem den Behälter 1 verschließenden Stöpsel 1' angeordnet sein. Der Lichtleiter 10 kann dabei durch den Stöpsel durchgeführt, bzw., wie aus Fig. 2b ersichtlich, von außen an einen optisch durchlässigen Stöpsel 1' herangeführt sein.

Bei einer weiteren, in Fig. 3 dargestellten Ausführungsvariante ist die Optode 3 an der Spitze einer Sonde 19 befestigt, welche das Ende des zweiarmigen Lichtleiters 10 aufnimmt. Die Sonde 19 wird durch die mit einem Septum 20 verschlossene Öffnung 21 des Behälters 1 in diesen eingeführt und kann entlang des Pfeiles 22 axial verschoben werden, sodaß Messungen sowohl im Gasraum 6 als auch in der Nährlösung 5 möglich sind.

Bei der in Fig. 4 dargestellten Ausführungsvariante ist die Optode 3, beispielsweise zur Messung von O₂ und CO₂ nicht im Behälter 1 sondern in einem Probennahmegefäß 23 angeordnet. Zur Einbringung der Probe in den Kulturbehälter wird das Septum 20 des Behälters 1 von der Hohlnadel 24 des Probennahmegefäßes 23 durchstochen, wodurch die Probe in die Nährlösung gelangt. Über die Hohlnadel 24 kommt es dann zu einem Gasaustausch zwischen Gasraum 6 und Innenraum 25 des Probennahmegefäßes 23, wodurch die Änderung der Konzentration von CO₂ und O₂ mittels der hier nicht weiter dargestellten Anregungs- und Detektionseinrichtung 8 erfaßbar ist.

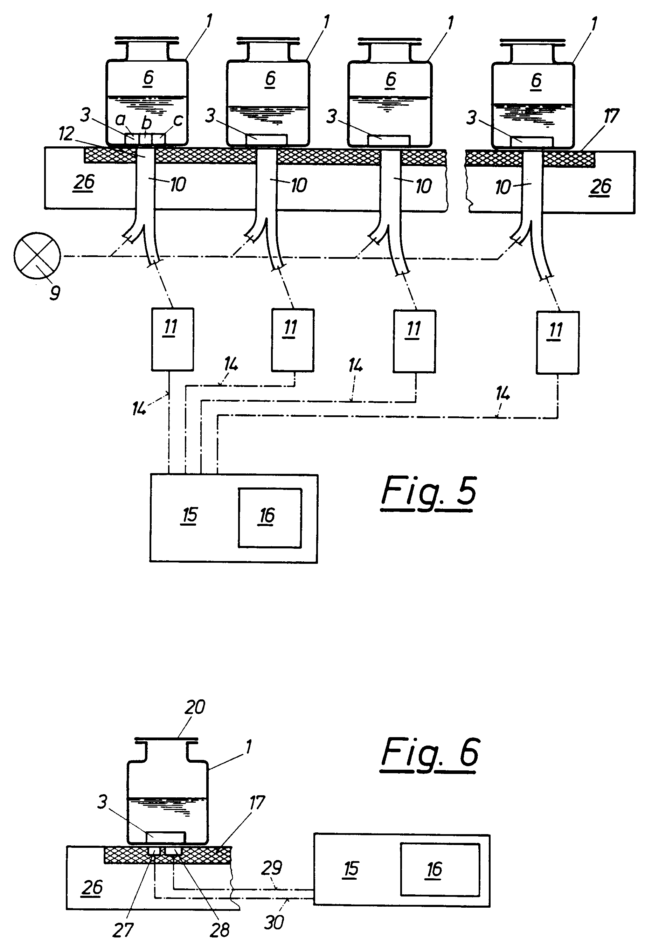

Eine besonders vorteilhafte Ausführungsvariante ist in Fig. 5 dargestellt, wo auf einer als thermostatisierbare Trägerplatte ausgeführte Einrichtung 26 mehrere Behälter 1 gleichzeitig in markierten Positionen aufgenommen werden können. Die Behälter 1 sind in mehreren Reihen angeordnet, sodaß gleichzeitig bis zu 600 Behälter thermostatisiert und kontinuierlich vermessen werden können. Jedem Behälter ist ein in der Trägerplatte 26 angeordneter zweiarmiger Lichtleiter 10 zugeordnet, der die am Boden jedes Behälters 1 angeordneten Optoden 3a bis 3c mit Anregungsstrahlung versorgt. Die entsprechenden optischen Signale werden den einzelnen Detektoren 11 zugeführt, welche mit der Auswerteeinrichtung 15 über Leitungen 14 verbunden sind. Zu den einzelnen Meßwerten wird der Auswerteeinheit 15 gleichzeitig ein Positionserkennungssignal zugeführt, wodurch die einzelnen Meßwerte direkt der entsprechenden Probe zuordenbar sind.

Eine Ausführungsvariante nach Fig. 6 sieht vor, daß jedem der einzelnen Behälter (1) eine direkt in der Trägerplatte 26 angeordnete LED 27 sowie eine Photodiode 28 zugeordnet ist, wobei auch Filterelemente vorgeschaltet sein können. Die Vorrichtung wird dadurch äußerst kompakt und enthält überhaupt keine beweglichen Teile oder Lichtleiteinrichtungen. Die elektrischen Anschlüsse der LED 27 bzw. der Photodiode 28 sind mit 30 und 29 bezeichnet.

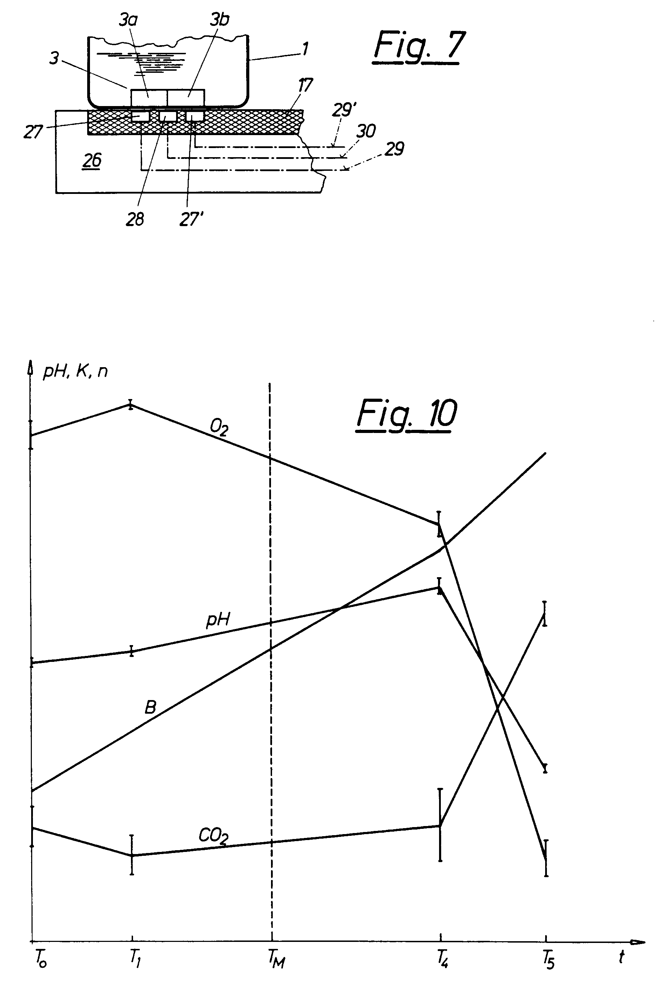

In Fig. 7 ist eine Ausführungsvariante nach Fig. 6 dargestellt, welche zwei zu einem Sensor zusammengefaßte Optoden 3a und 3b aufweist (z.B. ein Bisensor zur gleichzeitigen Messung der O₂-Konzentration und des pH-Wertes). Die Optoden werden über unterschiedliche LEDs 27 und 27' angeregt und deren Emissionsstrahlung von einer gemeinsamen Photodiode 28 erfaßt. Die entsprechenden elektrischen Anschlüsse 29, 29' und 30 führen zur hier nicht dargestellten Auswerteeinheit. Durch bekannte optische bzw. elektronische Einrichtungen können die Signale der beiden Optoden getrennt werden. Andere Ausführungen mit nur einer LED zur Anregung und mehreren Photodioden zur Signalerfassung liegen im Rahmen der Erfindung.

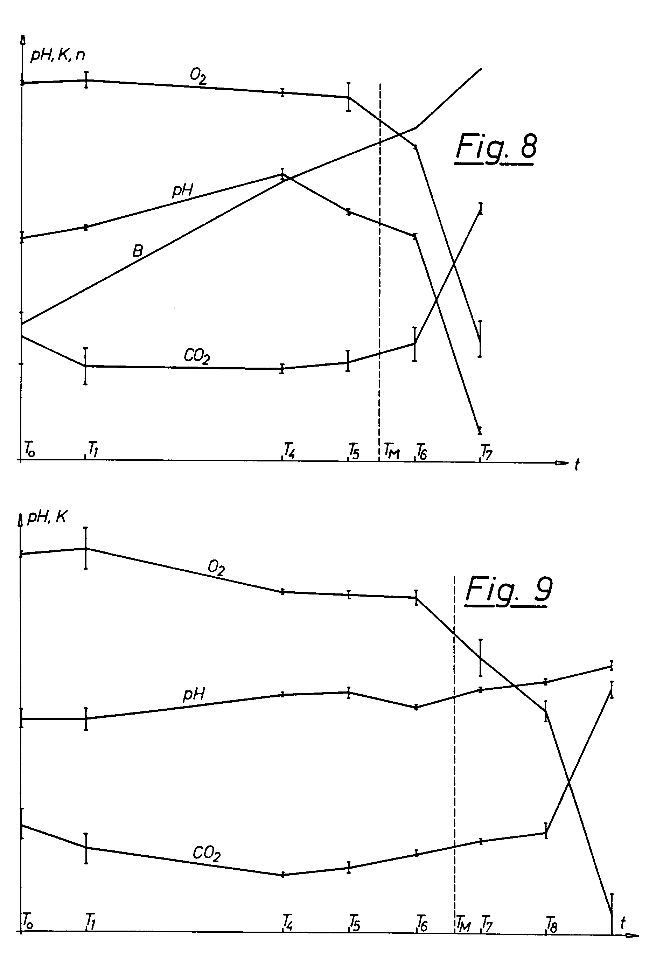

In den in den Fig. 8 bis 9 dargestellten Diagrammen von Meßbeispielen sind auf der Abszisse jeweils die Zeit t bzw. einzelne Zeitpunkte Tₒ bis T₈ aufgetragen und auf der Ordinate der pH-Wert, die Konzentration K (bzw. der Partialdruck von O₂ und CO₂), sowie die Anzahl n der Bakterien bzw. Organismen pro Volumseinheit (logarithmische Skala).

Fig. 8 zeigt die zeitliche Veränderung der Parameter O₂, CO₂ und pH-Wert anhand einer Probe mit Staphylokokkus aureus. Zwischen T₅ und T₆ steigt die CO₂-Konzentration signifikant an und zeigt damit eine positive Probe an, wonach zum Zeitpunkt Tₘ die O₂-Konzentration und der pH-Wert gemessen werden. Die stark fallende O₂-Konzentration und der stark fallende pH-Wert deuten auf grampositive Staphylokokkus aureus hin.

Zum Unterschied dazu bleibt in Fig. 9 der pH-Wert im wesentlichen unverändert, was entsprechend obenstehender Tabelle das Vorhandensein von Pseudomonas Spezies andeutet. Verwendet wurde hier eine Probe mit Pseudomonas aeruginosa.

Die in Fig. 10 angeführten Meßwerte stammen von einer Probe, die Enterobakterien (E.coli) enthält.

Das vorliegende Verfahren bzw. die beschriebene Vorrichtung eignet sich hervorragend zur Feststellung biologischer Aktivität in Proben, beispielsweise von Keimen in Blut, Bakteriaemien, Sepsis oder Pyämien aber auch von Algen, Bakterien oder anderen Keimen.

Durch das kontinuierliche, nichtinvasive Monitoring kann eine vollständige Automatisierung des Brut- und Meßvorganges für eine große Anzahl von Proben erreicht werden. Positive Kulturen werden rasch erkannt, wodurch falsche Negativbefunde weitgehend vermieden werden können.

## Patentansprüche

1. Verfahren zur Feststellung biologischer Aktivitäten in einer Probe, wobei die Probe und eine Nährlösung in einen verschließbaren Behälter gefüllt und Bedingungen ausgesetzt werden, welche bei Vorliegen von Mikroorganismen in der Probe metabolische Prozesse ermöglichen, wobei die Konzentration von Ausgangsstoffen vermindert und jene von Stoffwechselprodukten erhöht wird, **dadurch gekennzeichnet**, daß die Konzentration zumindest eines durch metabolische Prozesse umsetzbaren (gewonnenen oder verbrauchten) Stoffes kontinuierlich gemessen wird und bei Änderung der Konzentration um einen vorgebbaren Schwellwert die Konzentrationsänderung zumindest eines weiteren Stoffes gemessen wird, wobei mittels Optoden, welche in direktem Kontakt mit den zu messenden Stoffen stehen, ein Meßsignal erzeugt wird, aus dessen zeitlicher Änderung auf das Vorliegen von Mikroorganismen geschlossen wird.

2. Verfahren nach Anspruch 1, **dadurch gekennzeichnet**, daß die Konzentration zumindest zweier Stoffe aus der Gruppe CO₂, O₂, H⁺(pH), NH₄⁺, H₂S und H₂ gemessen wird, wobei die Indikatorsubstanz der Optoden auf eine Änderung der Konzentration der Stoffe mit einer Änderung ihres Lumineszenz-, Absorptions- oder Reflexionsverhaltens reagiert.

3. Verfahren nach Anspruch 1, **dadurch gekennzeichnet**, daß die Konzentration zumindest zweier Stoffe aus der Gruppe CO₂, O₂, H⁺(pH) und NH₄⁺ gemessen wird, wobei die Indikatorsubstanz der Optoden auf eine Änderung der Konzentration der Stoffe mit einer Änderung der Lumineszenzabklingzeit der emittierten Lumineszenzstrahlung reagiert.

4. Verfahren nach einem der Ansprüche 1 bis 3, **dadurch gekennzeichnet**, daß die CO₂-Konzentration kontinuierlich gemessen und daß nach deren Anstieg um 0,1 bis 10% über einen minimalen Wert die Änderung der O₂-Konzentration und die Änderung des pH-Wertes bestimmt wird.

5. Verfahren nach einem der Ansprüche 1 bis 3, **dadurch gekennzeichnet**, daß die O₂-Konzentration kontinuierlich gemessen und daß nach deren Abfall um 0,1 bis 10 % von einem maximalen Wert die Änderung der CO₂-Konzentration und die Änderung des pH-Wertes bestimmt wird.

6. Verfahren nach Anspruch 4 oder 5, **dadurch gekennzeichnet**, daß bei schwach fallender O₂-Konzentration und einem schwachen Absinken des pH-Wertes auf das Vorliegen von Enterobakterien oder auf Staphylokokkus epidermidis geschlossen wird.

7. Verfahren nach Anspruch 4 oder 5, **dadurch gekennzeichnet**, daßbei stark fallender O₂-Konzentration und stabilem pH-Wert auf das Vorliegen von Pseudomonas Spezies geschlossen wird.

8. Verfahren nach Anspruch 4 oder 5, **dadurch gekennzeichnet**, daß bei stark fallender O₂-Konzentration und schwach fallendem pH-Wert auf das Vorliegen von Acinetobacter Spezies geschlossen wird.

9. Verfahren nach Anspruch 4 oder 5, **dadurch gekennzeichnet**, daß bei unveränderter O₂-Konzentration und schwach fallendem pH-Wert auf bakteroide Spezies geschlossen wird.

10. Verfahren nach Anspruch 4 oder 5, **dadurch gekennzeichnet**, daß bei stark fallender O₂-Konzentration und stark fallendem pH-Wert auf Staphylokokkus aureus oder auf Streptokokkus faecalis geschlossen wird.

11. Verfahren nach Anspruch 4 oder 5, **dadurch gekennzeichnet**, daß bei schwach fallender O₂-Konzentration und stark fallendem pH-Wert auf das Vorliegen von Streptokokkus pyogenes, Streptokokkus pneumoniae oder Candida albicans geschlossen wird.

12. Verfahren nach Anspruch 4 oder 5, **dadurch gekennzeichnet**, daß bei gleichbleibender O₂-Konzentration und stark fallendem pH-Wert auf das Vorliegen von Chlostridium perfringens geschlossen wird.

13. Verfahren nach Anspruch 6, **dadurch gekennzeichnet**, daß zusätzlich die Gramfärbung der Probe festgestellt wird und bei negativer Gramfärbung auf das Vorliegen von Enterobakterien, bei positiver Gramfärbnung auf das Vorliegen von Staphylokokkus epidermidis geschlossen wird.

14. Vorrichtung zur Feststellung biologischer Aktivitäten in einer Probe, wobei ein verschließbarer Behälter vorgesehen ist, welcher eine Nährlösung enthält und zur Aufnahme der Probe dient, wobei Einrichtungen vorgesehen sind, die bei Vorliegen von Mikroorganismen in der Probe metabolische Prozesse ermöglichen, **dadurch gekennzeichnet**, daß mehrere Optoden (3a, 3b, 3c) innerhalb des verschließbaren Behälters (1) zur gleichzeitigen Bestimmung mehrerer durch den metabolischen Prozeß in deren Konzentration veränderbarer Stoffe vorgesehen sind, sowie daß eine jeder Optode (3a, 3b, 3c) zugeordnete Anregungs- und Detektionseinrichtung (8) vorgesehen ist, mit welcher eine Auswerteeinrichtung (15) zur Feststellung der zeitlichen Änderung der Konzentration der Stoffe verbunden ist.

15. Vorrichtung nach Anspruch 14, **dadurch gekennzeichnet**, daß Optoden (3a, 3b) zur selektiven Erfassung zumindest zweier im metabolischen Prozeß als Ausgangs-, Zwischen- oder Endprodukt vorliegender Stoffe aus der Gruppe CO₂, O₂, H₂, H⁺(pH), NH₄⁺ und H₂S vorhanden sind.

16. Vorrichtung nach Anspruch 15, **dadurch gekennzeichnet**, daß Optoden zur gleichzeitigen Bestimmung von CO₂, O₂ und H⁺(pH) vorhanden sind.

17. Vorrichtung nach Anspruch 15, **dadurch gekennzeichnet**, daß Optoden zur gleichzeitigen Bestimmung von CO₂, NH₄⁺ und H⁺(pH) vorhanden sind.

18. Vorrichtung nach Anspruch 15, **dadurch gekennzeichnet**, daß Optoden zur gleichzeitigen Bestimmung von CO₂, H₂S und H⁺(pH) vorhanden sind.

19. Vorrichtung nach einem der Ansprüche 15 bis 18, **dadurch gekennzeichnet**, daß die Optoden zu einem Multilayer-Sensor zusammengefaßt sind.

20. Vorrichtung nach Anspruch 14, **dadurch gekennzeichnet**, daß die Optoden (3a, 3b) im Gasraum (6) des zumindest teilweise optisch durchlässigen Behälters (1) über der mit der Probe versetzten Nährlösung angeordnet sind und die Änderung der Konzentration zumindest eines gasförmigen Metaboliten messen.

21. Vorrichtung nach Anspruch 20, **dadurch gekennzeichnet**, daß die Optoden (3a, 3b) an einem den Behälter (1) verschließenden, optisch durchlässigen Stöpsel (1') angeordnet sind.

22. Vorrichtung nach Anspruch 14, **dadurch gekennzeichnet**, daß eine Einrichtung (26) zur Thermostatisierung der Probe vorgesehen ist, in welcher in markierten Positionen mehrere Behälter (1) gleichzeitig angeordnet sind, wobei jedem der Behälter (1) eine Anregungs- und Detektionseinrichtung (8) zugeordnet ist, welche die in jedem Behälter (1) angeordneten Optoden (3a, 3b, 3c) mit Anregungsstrahlung versorgt und das resultierende optische Signal detektiert, sowie daß die Signale der Detektionseinrichtung samt einem Positionserkennungssignal der Auswerteeinrichtung (15) zuführbar sind.

23. Vorrichtung nach Anspruch 14, **dadurch gekennzeichnet**, daß eine Einrichtung (26) zur Thermostatisierung der Probe vorgesehen ist, welche mehrere Behälter (1) gleichzeitig aufnimmt, sowie daß ein Zuführmechanismus oder Probenwechsler vorgesehen ist, welcher die einzelnen Behälter (1) automatisch einem Meßplatz zuführt, in welchem die in jedem Behälter (1) angeordneten Optoden (3a, 3b, 3c) mit der Anregungs- und Detektionseinrichtung (8) in optischen Kontakt treten.

24. Vorrichtung nach Anspruch 14, **dadurch gekennzeichnet**, daß die Optoden an der Spitze einer in den Behälter (1) einführbaren Sonde (19) befestigbar sind, welche Sonde (19) Lichtleiteinrichtungen (10) der Anregungs- und Detektionseinrichtung (8) aufnimmt.

25. Vorrichtung nach Anspruch 24, **dadurch gekennzeichnet**, daß die Sonde (19) durch eine mit einem Septum (20) verschlossene Öffnung (21) des Behälters (1) in die mit der Probe versetzte Nährlösung oder in den Gasraum (6) darüber einbringbar ist.

26. Vorrichtung zur Feststellung biologischer Aktivitäten in einer Probe, wobei ein verschließbarer Behälter vorgesehen ist, welcher eine Nährlösung enthält und zur Aufnahme der Probe dient, wobei Einrichtungen vorgesehen sind, die bei Vorliegen von Mikroorganismen in der Probe metabolische Prozesse ermöglichen, **dadurch gekennzeichnet**, daß der Behälter (1) mit einem Septum (20) verschlossen ist, welches von der Hohlnadel (24) eines Probennahmegefäßes (23) durchstechbar ist, daß im Probennahmegefäß (23) Optoden (3a, 3b, 3c) zur gleichzeitigen Bestimmung mehrerer durch den metabolischen Prozeß in deren Konzentration veränderbarer Stoffe angeordnet sind, daß nach dem Einbringen der Probe in den die Nährlösung enthaltenden Behälter (1) über die Hohlnadel (24) des Probennahmegefäßes (23) eine Strömungsverbindung vom Gasraum (6) des Behälters (1) zu den Optoden (3a, 3b, 3c) besteht, sowie daß eine jeder Optode (3a, 3b, 3c) zugeordnete Anregungs- und Detektionseinrichtung (8) vorgesehen ist, mit welcher eine Auswerteeinrichtung (15) zur Feststellung der zeitlichen Änderung der Konzentration der Stoffe verbunden ist.

## Claims

1. A method for detecting biological activities in a specimen, where the specimen and a nutrient solution are filled into a sealable container and are subjected to conditions enabling metabolic processes to take place in the presence of microorganisms in the sample, the concentration of initial substances being lowered and that of metabolic products being raised, **wherein** the concentration of at least one (produced or consumed) substance subject to conversion by metabolic processes is measured continuously, and wherein the change in concentration of at least one other substance is measured in case of a concentration change in the first substance by a given threshold value, a test signal being generated by means of optodes in direct contact with the substances to be assessed, and the change over time of this signal serving as an indicator for the presence of microorganisms.

2. A method according to claim 1, **wherein** concentrations are measured of at least two substances from the group of CO₂, O₂, H⁺(pH), NH₄⁺, H₂S, H₂, the indicator medium of the optodes responding to a change in substance concentration by changing its luminescence, absorption or reflectance behaviour.

3. A method according to claim 1, **wherein** concentrations are measured of at least two substances from the group of CO₂, O₂, H⁺(pH), NH₄⁺, the indicator medium of the optodes responding to a change in substance concentration by changing the luminescence decay time of the luminescent radiation emitted.

4. A method according to any of claims 1 to 3, **wherein** the CO₂ concentration is measured continuously and the changes in O₂ concentration and pH are determined upon an increase of the CO₂ concentration by 0.1 to 10% above a given minimum.

5. A method according to any of claims 1 to 3, **wherein** the O₂ concentration is measured continuously and the changes in CO₂ concentration and pH are determined upon a decrease of the O₂ concentration by 0.1 to 10% below a given maximum.

6. A method according to claim 4 or 5, **wherein** a slight decrease in both O₂ concentration and pH indicates the presence of enterobacteria or Staphylococcus epidermidis.

7. A method according to claim 4 or 5, **wherein** a sharp decrease in O₂ concentration and a stable pH indicate the presence of Pseudomonas species.

8. A method according to claim 4 or 5, **wherein** a sharp decrease in O₂ concentration and a slight decrease in pH indicate the presence of Acinetobacter species.

9. A method according to claim 4 or 5, **wherein** no change in O₂ concentration and a slight decrease in pH indicate the presence of bacteroid species.

10. A method according to claim 4 or 5, **wherein** a sharp decrease in both O₂ concentration and pH indicates the presence of Staphylococcus aureus or Streptococcus faecalis.

11. A method according to claim 4 or 5, **wherein** a slight decrease in O₂ concentration and a sharp decrease in pH indicate the presence of Streptococcus pyogenes, Streptococcus pneumoniae or Candida albicans.

12. A method according to claim 4 or 5, **wherein** no change in O₂ concentration and a sharp decrease in pH indicate the presence of Clostridium perfringens.

13. A method according to claim 6, **wherein** Gram staining of the sample is additionally determined, negative Gram staining indicating the presence of enterobacteriae, and positive Gram staining that of Staphylococcus epidermidis.

14. A device for detecting biological activities in a specimen, comprising a sealable container containing a nutrient solution into which the sample is introduced, and further comprising means enabling metabolic processes to take place in the presence of microorganisms in the sample, **wherein** several optodes (3a, 3b, 3c) inside the sealable container (1) are provided for simultaneous assessment of several substances whose concentration is subject to changes by the metabolic process, and wherein an excitation and detection assembly (8) is provided, which is assigned to each optode (3a, 3b, 3c), and which in turn is connected with an evaluation unit (15) determining the change over time of the substance concentration.

15. A device according to claim 14, **wherein** optodes (3a, 3b) are provided for selective detection of at least two substances from the group of CO₂, O₂, H₂, H⁺(pH), NH₄⁺ and H₂S, that are present during the metabolic process as initial, intermediate or final products.

16. A device according to claim 15, **wherein** optodes are provided for simultaneously determining CO₂, O₂ and H⁺(pH).

17. A device according to claim 15, **wherein** optodes are provided for simultaneously determining CO₂, NH₄⁺ and H⁺(pH).

18. A device according to claim 15, **wherein** optodes are provided for simultaneously determining CO₂, H₂S and H⁺(pH).

19. A device according to any of claims 15 to 18, **wherein** the optodes are combined to form a multilayer sensor.

20. A device according to claim 16, **wherein** the optodes (3a, 3b) are placed in the gas space (6) of the, at least partly, transparent container (1) above the nutrient solution mixed with the sample, and wherein they are used for measuring the concentration of at least one gaseous metabolite.

21. A device according to claim 20, **wherein** the optodes (3a, 3b) are located on a transparent stopper (1') used for sealing the container (1).

22. A device according to claim 14, **wherein** device (26) is provided for thermostat control of the sample, in which several containers (1) are placed in labelled positions at the same time, each container (1) being assigned an excitation and detection assembly (8) transmitting excitation radiation to the optodes (3a, 3b, 3c) located in each container (1) and detecting the ensuing optical signal, and wherein the signals of the detecion assembly are carried to the evaluation unit (15) together with a position identification signal.

23. A device according to claim 14, **wherein** a device (26) is provided for thermostat control of the sample, which holds several containers (1) at the same time, and wherein a feed mechanism or sample changer is provided, automatically taking the individual containers (1) to a measuring station, in which the optodes (3a, 3b, 3c) located in each container (1) enter into optical contact with the excitation and detection assembly (8).

24. A device according to claim 14, **wherein** the optodes are fastened at the tip of a probe (19) to be inserted into the container (1), which probe (19) contains lightguide elements (10) of the excitation and detection assembly (8).

25. A device according to claim 24, **wherein** the probe (19) is inserted through an opening (21) of the container (1) sealed by a septum (20) and introduced into the nutrient solution mixed with the sample, or into the gas space (6) above it.

26. A device for detecting biological activities in a specimen, comprising a sealable container containing a nutrient solution into which the sample is introduced, and further comprising means enabling metabolic processes to take place in the presence of microorganisms in the sample, **wherein** the container (1) is sealed with a septum (20), which can be punctured with the hollow needle (24) of a sampling vessel (23), and wherein optodes (3a, 3b, 3c) are located in the sampling vessel (23) for the simultaneous determination of several substances whose concentration is subject to change by the metabolic process, and wherein a flow connection is established between the gas space (6) of the container (1) and the optodes (3a, 3b, 3c) via the hollow needle (24) of the sampling vessel (23), after the sample has been added to the nutrient solution in the container (1), and, further, wherein an excitation and detection assembly (8) is provided, which is assigned to each optode (3a, 3b, 3c) and to which is connected an evaluation unit (15) determining the change over time of the substance concentration.

## Revendications

1. Procédé pour déterminer des activités biologiques dans un échantillon, en plaçant l'échantillon et une solution nutritive dans un récipient scellable et en les exposant à des conditions dans lesquelles, en cas de présence de micro-organismes dans l'échantillon, ils peuvent développer des processus métaboliques, la concentration des produits de départ diminuant et celle des produits résultant de l'échange de matière augmentant, caractérisé en ce qu'on dose en continu la concentration d'au moins une substance transformable (obtenue ou consommée) par des processus métaboliques, et en ce qu'on dose la variation de concentration d'une autre substance, en cas de modification de la première concentration au-delà d'une valeur seuil fixée, des optodes qui sont en contact direct avec les substances à doser donnant un signal de mesure dont l'évolution en fonction du temps permet de tirer des conclusions sur la présence des micro-organismes.

2. Procédé selon la revendication 1, caractérisé en ce qu'on mesure la concentration d'au moins deux substances du groupe CO₂, O₂, H⁺ (pH), NH₄⁺, H₂S et H₂, la substance indicatrice des optodes réagissant à une modification de la concentration des substances par une modification de son comportement de luminescence, d'absorption ou de réflexion.

3. Procédé selon la revendication 1, caractérisé en ce qu'on mesure la concentration d'au moins deux substances du groupe CO₂, O₂, H⁺ (pH) et NH₄⁺, la substance indicatrice des optodes réagissant à une modification de concentration des substances par une modification de la durée de diminution de luminescence du rayonnement de luminescence émis.

4. Procédé selon l'une des revendications 1 à 3, caractérisé en ce qu'on dose en continu la concentration en CO₂ et qu'après son augmentation de 0,1 à 10 % au-dessus d'une valeur minimale, on mesure la variation de la concentration en O₂ et la variation du pH.

5. Procédé selon l'une des revendications 1 à 3, caractérisé en ce qu'on dose en continu la concentration en O₂ et qu'après sa diminution de 0,1 à 10 % en-dessous d'une valeur maximale, on détermine la variation de la concentration de CO₂ et la variation du pH.

6. Procédé selon la revendication 4 ou 5, caractérisé en ce que lorsque la concentration en O₂ diminue faiblement et que le pH diminue faiblement on conclut à la présence d'entérobactéries ou de staphylococcus épidermidis.

7. Procédé selon la revendication 4 ou 5, caractérisé en ce que, lorsque la concentration d'O₂ chute fortement et que le pH reste stable, on conclut à la présence d'espèces pseudomonas.

8. Procédé selon la revendication 4 ou 5, caractérisé en ce qu'à forte chute de concentration en O₂ et faible diminution de pH, on conclut à la présence d'espèces acinétobacter.

9. Procédé selon la revendication 4 ou 5, caractérisé en ce que lorsque la concentration en O₂ ne varie pas et que le pH diminue faiblement, on conclut à la présence d'espèces bactéroïdes.

10. Procédé selon la revendication 4 ou 5, caractérisé en ce que lorsque la concentration en O₂ diminue fortement et que le pH chute fortement, on conclut à la présence de staphylococcus aureus ou de streptococcus faecalis.

11. Procédé selon la revendication 4 ou 5, caractérisé en ce que lorsque la concentration en O₂ diminue faiblement et que le pH chute fortement, on conclut à la présence de streptococcus pyogènes, streptococcus pneumoniae ou candida albicans.

12. Procédé selon la revendication 4 ou 5, caractérisé en ce qu'à concentration en O₂ constante et à pH chutant fortement, on conclut à la présence de chlostridium perfringens.

13. Procédé selon la revendication 6, caractérisé en ce qu'on établit en plus la coloration gram de l'échantillon et qu'en cas de coloration gram négative, on conclut à la présence d'entérobactéries, tandis qu'en cas de coloration gram positive, on conclut à la présence de staphylococcus épidermidis.

14. Dispositif pour déterminer des activités biologiques dans un échantillon en prévoyant un récipient scellable qui contient une solution nutritive servant à recevoir l'échantillon, en prévoyant des dispositifs qui permettent des processus métaboliques dans l'échantillon en présence de micro-organismes, caractérisé en ce qu'on prévoit plusieurs optodes (3a, 3b, 3c) pour doser simultanément plusieurs substances de concentrations variable à cause du processus métabolique, et en ce qu'on prévoit aussi un dispositif (8) d'excitation et de détection affecté à chaque optode (3a, 3b, 3c), auquel on relie un dispositif d'exploitation (15) pour mettre en évidence la modification de la concentration dans le temps des substances.

15. Dispositif selon la revendication 14, caractérisé en ce qu'on utilise des optodes (3a, 3b) pour capter sélectivement au moins deux substances présentes dans le processus métabolique comme produit de départ, produit intermédiaire ou produit final, des substances du groupe CO₂, O₂, H₂, H⁺ (pH), NH⁺₄ et H₂S.

16. Dispositif selon la revendication 15, caractérisé en ce qu'on utilise des optodes pour doser simultanément CO₂, O₂ et H⁺ (pH).

17. Dispositif selon la revendication 15, caractérisé en ce qu'on utilise des optodes pour doser simultanément CO₂, NH⁺₄ et H⁺ (pH).

18. Dispositif selon la revendication 15, caractérisé en ce qu'on utilise des optodes pour doser simultanément CO₂, H₂S et H⁺ (pH).

19. Dispositif selon l'une des revendications 15 à 18, caractérisé en ce que les optodes appartiennent à un capteur multicouche.

20. Dispositif selon la revendication 14, caractérisé en ce qu'on place les optodes (3a, 3b) dans l'espace gazeux (6) du récipient (1) au moins partiellement transparent optiquement, au-dessus de la solution nutritive comportant l'échantillon, et en ce qu'on mesure la modification de la concentration d'au moins un métabolite gazeux.

21. Dispositif selon la revendication 20, caractérisé en ce qu'on place les optodes (3a, 3b) sur un bouchon (1') optiquement transparent bouchant le récipient (1).

22. Dispositif selon la revendication 14, caractérisé en ce qu'on prévoit un dispositif (26) de thermorégulation de l'échantillon, dans lequel on dispose simultanément plusieurs récipients (1) dans des positions repérées, en attribuant à chacun des récipients (1) un dispositif d'excitation et de détection (8) qui alimente en rayonnement d'excitation les optodes (3a, 3b, 3c) placées dans chaque récipient (1), et détecte le signal optique résultant, et en ce qu'on peut conduire tous les signaux du dispositif de détection au dispositif d'exploitation (15) par un signal de reconnaissance de position.

23. Dispositif selon la revendication 14, caractérisé en ce qu'on prévoit un dispositif (26) de thermorégulation de l'échantillon qui reçoit plusieurs récipients (1) simultanément, et en ce qu'on prévoit un mécanisme de transfert ou un changeur d'échantillon qui conduit automatiquement chaque récipient (1) à un poste de mesure dans lequel les optodes (3a, 3b, 3c) placées dans chaque récipient (1) entrent en contact optique avec le dispositif d'excitation et de détection (8).

24. Dispositif selon la revendication 14, caractérisé en ce qu'on peut fixer les optodes au sommet d'une sonde (19) insérable dans le récipient (1), cette sonde (19) accueillant des dispositifs (10) guides de lumière de l'unité (8) d'excitation et de détection.

25. Dispositif selon la revendication 24, caractérisé en ce qu'on peut insérer la sonde (19) à travers un orifice (21) du récipient (1) fermé par un septum (20), dans la solution nutritive additionnée de l'échantillon ou dans l'espace gazeux (6) situé au-dessus.

26. Dispositif pour déterminer des activités biologiques dans un échantillon en prévoyant un récipient scellable, qui contient une solution nutritive servant à recevoir l'échantillon, en prévoyant des dispositifs qui permettent des processus métaboliques dans l'échantillon en présence de micro-organismes, caractérisé en ce qu'on ferme le récipient (1) par un septum (20) qu'on peut transpercer par l'aiguille creuse (24) d'un récipient de prélèvement d'échantillon (23), les optodes (3a, 3b, 3c) étant placées dans le récipient de prélèvement d'échantillon (23), pour doser simultanément plusieurs substances à concentration variable formées par le processus métabolique et après incorporation de l'échantillon dans le récipient (1) contenant la solution nutritive, au moyen de l'aiguille creuse (24) du récipient de prélèvement d'échantillon (23), en ce qu'on réalise une liaison de courant de l'espace gazeux (6) du récipient (1) vers les optodes (3a, 3b, 3c), et en ce qu'on prévoit pour chaque optode (3a, 3b, 3c) un dispositif d'excitation et de détection (8) auquel est relié un dispositif d'exploitation (15) pour déterminer la variation de la concentration des substances en fonction du temps.
